# NEW EUROPEAN PATENT SPECIFICATION

(11) **EP 1 969 127 B2**
(45) Date of publication and mention of the opposition decision: **12.07.2017**
(45) Mention of the grant of the patent: 18.06.2014
(21) Application number: 06848864.2
(22) Date of filing: 21.12.2006
(51) Int. Cl.: C12N 15/57, C12P 21/02

(54) **METHOD OF PRODUCING BIOLOGICALLY ACTIVE VITAMIN K DEPENDENT PROTEINS BY RECOMBINANT METHODS**
VERFAHREN ZUR HERSTELLUNG BIOLOGISCH WIRKSAMER VITAMIN-K-ABHÄNGIGER PROTEINE MIT REKOMBINANTEN VERFAHREN
PROCÉDÉ DE PRODUCTION DE PROTÉINES DÉPENDANTES DE LA VITAMINE K BIOLOGIQUEMENT ACTIVES PAR DES PROCÉDÉS RECOMBINANTS

(30) Priority: 21.12.2005 US 752642 P
(43) Date of publication of application: 17.09.2008
(60) Divisional application: 11157194.9
(73) Proprietor: CNJ HOLDINGS, INC, Winnipeg, MB R3T 5Y3 (CA); University of North Carolina at Chapel Hill, Chapel Hill, NC 27599 (US)
(72) Inventor: DROHAN, William, N., Springfield, VA 22152 (US); GRIFFITH, Michael, J., San Juan Capistrano, California 92675 (US); TAYLOR, John, R., New York, New York 10128 (US); STAFFORD, Darrel, W., Carrboro, North Carolina 27599 (US)
(74) Representative: Gill Jennings & Every LLP
(86) International application number: PCT/US2006/048954
(87) International publication number: WO 2007/075976

(56) References cited:
- EP-A2- 2 385 125
- WO-A-02/29045
- WO-A-92/01795
- WO-A-92/09698
- WO-A-92/19636
- WO-A-96/34966
- WO-A-2005/030039
- WO-A-2005/038019
- WO-A-2006/067116
- WO-A-2006/089613
- WO-A-2006/101474
- WO-A-2006/110083
- WO-A2-2005/030039
- US-A- 5 888 809
- US-A- 5 888 809
- US-A1- 2006 121 574
- WAJIH N ET AL: "Increased production of functional recombinant human clotting factor IX by baby hamster kidney cells engineered to overexpress VKORC1, the vitamin K 2,3-epoxide-reducing enzyme of the vitamin K cycle" JOURNAL OF BIOLOGICAL CHEMISTRY, AMERICAN SOCIETY OF BIOLOCHEMICAL BIOLOGISTS, BIRMINGHAM,, US, vol. 280, no. 36, 19 July 2005 (2005-07-19), pages 31603-31607, XP002375182 ISSN: 0021-9258
- SUN YAN-MEI ET AL: "Vitamin K epoxide reductase significantly improves carboxylation in a cell line overexpressing factor X" BLOOD, W.B.SAUNDERS COMPANY, ORLANDO, FL, US, vol. 106, no. 12, 4 August 2005 (2005-08-04), pages 3811-3815, XP002377556 ISSN: 0006-4971
- WASLEY L C ET AL: "PACE/FURIN CAN PROCESS THE VITAMIN K-DEPENDENT PRO-FACTOR IX PRECURSOR WITHIN THE SECRETORY PATHWAY" JOURNAL OF BIOLOGICAL CHEMISTRY, AMERICAN SOCIETY OF BIOLOCHEMICAL BIOLOGISTS, BIRMINGHAM,, US, vol. 268, no. 12, 25 April 1993 (1993-04-25), pages 8458-8465, XP001094106 ISSN: 0021-9258
- REHEMTULLA A ET AL: "In vitro and in vivo functional characterization of bovine vitamin K-dependent gamma-carboxylase expressed in Chinese hamster ovary cells" PROCEEDINGS OF THE NATIONAL ACADEMY OF SCIENCES OF USA, NATIONAL ACADEMY OF SCIENCE, WASHINGTON, DC, US, vol. 90, no. 10, May 1993 (1993-05), pages 4611-4615, XP002204115 ISSN: 0027-8424
- WAJIH NADEEM ET AL: "Engineering of a recombinant vitamin K-dependent gamma-carboxylation system with enhanced gamma-carboxyglutamic acid forming capacity - Evidence for a functional CXXC redox center in the system" JOURNAL OF BIOLOGICAL CHEMISTRY, AMERICAN SOCIETY OF BIOLOCHEMICAL BIOLOGISTS, BIRMINGHAM,, US, vol. 280, no. 11, 7 January 2005 (2005-01-07), pages 10540-10547, XP002377554 ISSN: 0021-9258
- ROTH DAVID A ET AL: "Human recombinant factor IX: Safety and efficacy studies in hemophilia B patients previously treated with plasma-derived factor IX concentrates" BLOOD, vol. 98, no. 13, 15 December 2001 (2001-12-15), pages 3600-3606, XP002442457 ISSN: 0006-4971
- RUNNING DEER JENNIFER ET AL: "High-level expression of proteins in mammalian cells using transcription regulatory sequences from the Chinese hamster EF-1alpha gene", BIOTECHNOLOGY PROGRESS, AMERICAN INSTITUTE OF CHEMICAL ENGINEERS, US, vol. 20, no. 3, 10 March 2004 (2004-03-10) , pages 880-889, XP002557337, ISSN: 8756-7938, DOI: 10.1021/BP034383R [retrieved on 2008-09-05]
- KAUFMAN R J ET AL: "EXPRESSION PURIFICATION AND CHARACTERIZATION OF RECOMBINANT GAMMA CARBOXYLATED FACTOR-IX SYNTHESIZED IN CHINESE HAMSTER OVARY CELLS", JOURNAL OF BIOLOGICAL CHEMISTRY.(MICROFILMS), AMERICAN SOCIETY OF BIOLOGICAL CHEMISTS, BALTIMORE, MD, US, vol. 261, no. 21, 25 July 1986 (1986-07-25), pages 9622-9628, XP002375183,
- TERUYA K. ET AL: 'An approach to further enhance the cellular productivity of exogenous protein hyper-producing Chinese hamster ovary (CHO) cells' CYTOTECHNOLOGY vol. 47, 2005, pages 29 - 36
- WAJIH N. ET AL: 'The inhibitory effect of calumenin on the vitamin K-dependent y-carboxylation stistem' THE JOURNAL OF BIOLOGICAL CHEMISTRY vol. 279, no. 24, 2004, pages 25276 - 25283
- WALSH G. ET AL: 'Post-translational modifications in the context of therapeutic proteins' NATURE BIOTECHNOLOGY vol. 24, no. 10, October 2006, pages 1241 - 1252
- RUNNING DEERE J. ET AL: 'High-level expression of proteins in mammalian cells using transcription regulatory sequences from the Chinese hamster EF-1a gene' BIOTECHNOL. PROG. vol. 20, 2004, pages 880 - 889
- GGCX (P38435), Molecular Target Synopsis, downloaded Feb. 25, 2015
- NAKAI H. ET AL: 'Adeno-associated viral vector-mediated gene transfer of human blood coagulation factor IX into mouse liver' BLOOD vol. 91, no. 12, 15 June 1998, pages 4600 - 4607
- KAUFMAN R.J. ET AL: 'Expression, purification, and characterization of recombinant y-carboxylated factor IX synthesized in Chinese hamster ovary cells' THE JOURNAL OF BIOLOGICAL CHEMISTRY vol. 261, no. 21, 1986, pages 9622 - 9628
- CLS Behring/Coagulation Factor IX (Human) Mononine®
- Mononine 1000 IU (https://www.medicines.org.uk/emc/medicine/ 14372), downloaded on March 17, 2015

## Description

### BACKGROUND OF THE INVENTION

### Field of the Invention

Embodiments of the invention relate generally to production of recombinant vitamin K dependent proteins, particularly Factor IX, which are fully functional by co-expression of one or more proteins involved in the processing of the vitamin K dependent proteins. These processing proteins include, vitamin K dependent epoxide reductase (VKOR) and vitamin K dependent γ-glutamyl carboxylase (VKGC). Additionally, the propeptide of the vitamin K dependent protein may be modified to improve γ-carboxylation.

### Description of the Related Art

Bleeding disorders can result from a deficiency in the functional levels of one or more of the blood proteins, collectively known as blood coagulation factors, that are required for normal hemostasis, i.e. blood coagulation. The severity of a given bleeding disorder is dependent on the blood level of functional coagulation factors. Mild bleeding disorders are generally observed when the functional level of a given coagulation factor reaches about 5% of normal, but if the functional level falls below 1%, severe bleeding is likely to occur with any injury to the vasculature.

Medical experience has shown that essentially normal hemostasis can be temporarily restored by intravenous infusion of biological preparations containing one or more of the blood coagulation factors. So-called replacement therapy, whereby a biological preparation containing the deficient blood coagulation factor is infused when bleeding occurs (on demand) or to prevent bleeding (prophylactically), has been shown to be effective in managing patients with a wide variety of bleeding disorders. In general, for replacement therapy to be effective, intravenous infusions of the missing coagulation factor are targeted to achieve levels that are well above 5% of normal over a two- to three-day period.

Historically, patients who suffer from hemophilia, a genetically acquired bleeding disorder that results from a deficiency in either blood coagulation Factor VIII (hemophilia A) or Factor IX (hemophilia B), were successfully treated by periodic infusion of whole blood or blood plasma fractions of varying degrees of purity.

More recently, with the advent of biotechnology, biologically active preparations of synthetic (recombinant) blood coagulation factors have become commercially available for treatment of blood coagulation disorders. Recombinant blood coagulation proteins are essentially free of the risks of human pathogen contamination that continue to be a concern that is associated with even high purity commercial preparations that are derived from human blood.

Adequate treatment of bleeding disorders is largely limited to the economically-developed regions of the world. In the case of hemophilia it is estimated that over 75% of the patient population worldwide receives inadequate or, worse, no treatment of their disease. For many regions of the world, the cost of safe and effective commercial preparations of coagulation factors is prohibitive for routine management of bleeding disorders and, in some cases, only emergency treatment with donated products is available.

In regions of the world where adequate treatment of bleeding disorders is potentially available, the cost is very high and patients are almost always dependent on third party payors, e.g. health insurance or government subsidized programs, to acquire the commercial products needed. On average, hemophilia treatment in the United States is estimated to cost about $50,000 per patient per year for the commercial product required for routine, on-demand, care. However, this cost could be much higher insofar as the Medical and Scientific Advisory Committee for the National Hemophilia Foundation has recommended that patients should receive prophylactic treatment which, in the case of an adult hemophiliac, could drive the annual cost to well over $250,000 per year. Given that life-time insurance caps of about $1 million are generally associated with most policies in the United States, hemophiliacs are severely constrained in terms of the amount of commercial product that they can afford for care which, at the least, affects their quality of life during adulthood and, at the worst, raises the risk of life-threatening bleeding.

For the past 25 years or so, biotechnology has offered the promise of producing low cost biopharmaceutical products. Unfortunately, this promise has not been met due in major part to the inherent complexity of naturally occurring biological molecules and a variety of limitations associated with the synthesis of their recombinant protein counterparts in genetically engineered cells. Regardless of the cell type, e.g. animal, bacteria, yeast, insect, plant, etc., that is chosen for synthesis, proteins must achieve certain minimal structural properties for safe and effective therapeutic use. In some cases, recombinant proteins must simply fold correctly after synthesis to attain the three-dimensional structure required for proper function. In other cases, recombinant proteins must undergo extensive, enzyme directed, post-translational modification after the core protein has been synthesized within the cell. Deficiencies in any one of a number of intracellular enzymatic activities can result in the formation of a large percentage of non-functional protein and limit the usefulness of a genetically engineered cell system for the economical production of a biopharmaceutical product intended for commercial use.

Several of the proteins required for normal blood coagulation are very complex in terms of having multiple structural domains each being associated with a very specific functional property that is essential for the overall effectiveness of the protein in controlling hemostasis and/or preventing thrombosis. In particular, the so-called "vitamin K-dependent" blood coagulation proteins, e.g. Factors II, VII, IX, X, Protein C and Protein S, are very complex proteins and must undergo extensive post-translational modification for normal function. Achieving high levels of functional vitamin K-dependent proteins by recombinant technology has been limited by the structural complexity of these proteins and the inability to create genetically engineered cell systems that overcome the inherent deficiencies in the enzymatic activities required for efficient and complete post-translational modification to occur.

### Problem to be solved

The first synthetic vitamin K-dependent blood coagulation protein to become commercially available was Factor IX which is still manufactured today from genetically engineered Chinese Hamster Ovary (CHO) cells (BeneFix, Coagulation Factor IX (Recombinant) Directional Insert, Weyth Pharmaceuticals, Inc. Philadelphia, PA 19101 CI 8020-3 W10483C007, Rev 10/05). Although recombinant Factor IX can be produced using CHO cells, it is not optimal as a treatment for Hemophilia B because it has not been properly processed and consequently its bioavailability to patients is variable. While reasonable levels of recombinant Factor IX protein can be expressed by genetically engineered CHO cells, e.g. up to 188 mg/L, the levels of fully functional Factor IX that are produced are on the order of only 0.5 mg/L due to the limited ability of the CHO cells to fully gamma-carboxylate the first 12 glutamic acid residues in the amino terminal region of the protein referred to as the gladomain. In addition to this deficiency in the post-translational modification of Factor IX, subsequent work demonstrated that pro-Factor IX, a form of Factor IX that contains a propeptide domain that is required for the efficient intracellular gamma-carboxylation of the protein, is not processed completely prior to secretion from the CHO cell. As a consequence, it was found that well over half of the Factor IX secreted from genetically engineered CHO cells still contains the propeptide region and is non-functional (Bond, M., Jankowski, M., Patel, H., Karnik, S., Strand, A., Xu, B., et al. [1998] Biochemical characterization of recombinant factor IX. Semin. Hematol. 35 [2 Suppl.2], 11-17).

The present application addresses a need for a method to produce vitamin K dependent proteins such as Factor IX which have been properly processed so that they are active and in sufficient yield for commercial production. To increase the availability of vitamin K-dependent blood coagulation proteins to meet the worldwide medical need for the treatment of bleeding disorders such as hemophilia B, improvements in the production of fully functional protein, Factor IX in this example, from genetically engineered cells are required. Specifically, identification and supplementation of deficiencies in the enzymatic activities required to obtain essentially complete post-translational modification are needed.

### SUMMARY OF THE INVENTION

According to the present invention there is provided a method of producing recombinant biologically active Factor IX protein product as set out in claim 1.

Embodiments of the invention are directed to methods of producing a recombinant biologically active Factor IX product, which includes transfecting a CHO cell with a gene encoding Factor IX protein operably linked to a promoter and at least two genes encoding a processing factor(s) operably linked to at least one promoter, either simultaneously or sequentially, and harvesting the Factor IX protein product. Preferably, the cell produces biologically active vitamin K dependent protein product in an amount of at least about 15 mg/L.

The processing factor is a nucleic acid selected from, vitamin K dependent epoxide reductase (VKOR) and vitamin K dependent γ-glutamyl carboxylase (VKGC) operably linked to one or more promoter(s). Preferably, the one or more processing factor proteins is produced in an amount sufficient to facilitate the production of at least about 15 mg/L of the recombinant biologically active vitamin K dependent protein product. Preferably, at least one of the genes is overexpressed. More preferably, the overexpressed gene is operably linked to a Chinese hamster elongation factor 1-α (CHEF1) promoter.

In preferred embodiments, at least about 75% of the glutamic acid residues within the gla-domain of the biologically active Factor IX protein product are gamma carboxylated.

In some preferred embodiments, the Factor IX protein product has a deletion in a propeptide of the Factor IX protein product.

In some preferred embodiments, the Factor IX protein product includes a heterologous propeptide region which is from a Factor IX protein which is different from the Factor IX protein product.

Preferably, at least 10% of the recombinant Factor IX protein is biologically active. More preferably, at least 20% of the Factor IX protein is biologically active. Yet more preferably, at least 50% of the Factor IX protein is biologically active. Yet more preferably, at least 80% of the Factor IX protein is biologically active.

In preferred embodiments, the biologically active Factor IX protein is produced in an amount of at least about 20 mg/L. More preferably, the biologically active Factor IX protein is produced in an amount of at least about 30 mg/L. More preferably, the biologically active Factor IX protein is produced in an amount of at least about 50 mg/L.

In some preferred embodiments, transfection is sequential and transfecting the mammalian cell further includes selecting for cells which express high levels of the Factor IX protein product or the processing factor(s), cloning the selected cells, and amplifying the cloned cells. In some preferred embodiments, the transfecting steps with the gene(s) encoding the processing factor(s) are performed before the transfecting steps with the gene encoding the Factor IX protein. In alternate preferred embodiments, the transfecting steps with the gene encoding the Factor IX protein are performed before transfecting steps with the gene(s) encoding the processing factor(s).

In preferred embodiments, the CHO cell is selected for expression of endogenous levels of one or more processing factors before transfection.

Embodiments of the invention are directed to a recombinant CHO cell which includes a gene for Factor IX protein operably linked to a promoter and a gene for at least one processing factor operably linked to at least one promoter. The expression of the protein(s) encoded by the gene for at least one processing factor(s) in the cell facilitates the production of biologically active Factor IX protein in an amount of preferably at least about 15 mg/L.

The processing factor is a gene which produces a processing gene product selected from VKOR and VKGC, operably linked to one or more promoter(s) for expression in said cell. Preferably, the at least one processing gene products is expressed at a higher level than observed in normal, nontransfected cells of the same line. More preferably, the overexpressed gene product is operably linked to a Chinese hamster elongation factor I-α (CHEF1) promoter.

In preferred embodiments, the gene encoding the Factor IX protein is modified to increase the percentage of glutamic acid residues which are carboxylated when compared to the percentage of carboxylated glutamic acid residues present on Factor IX protein produced from cells expressing a Factor IX protein encoded by a gene encoding the unmodified Factor IX protein.

In some preferred embodiments, the modification includes a deletion in the propeptide region of the gene encoding the Factor IX protein.

In some preferred embodiments, the modification includes substitution of a propeptide region of the Factor IX protein with a heterologous propeptide region from a heterologous Factor IX protein.

In some preferred embodiments, the cell used for transfection of a gene for a Factor IX protein is preselected by selecting for variants of a specific tissue culture cell line that contain naturally occurring modification enzymes capable of producing a Factor IX protein composed of amino acids that are posttranslationally modified to contain at least 25% of the sulfation and at least 25% of the phosphorylation levels present in the corresponding plasma-derived Factor IX protein.

In preferred embodiments, a recombinant Factor IX protein is produced by one or more of the method steps described herein. The recombinant Factor IX protein produced by the methods described may be included in a pharmaceutical composition, or a kit. The recombinant Factor IX protein may be used in a method of treating hemophilia by administering an effective amount of the recombinant Factor IX protein to a patient in need thereof.

Preferred embodiments are directed to methods of producing recombinant biologically active Factor IX protein products, by a process involving one or more of the following steps:
(a) transfecting a mammalian cell with a gene encoding the Factor IX protein operably linked to a promoter;
(b) selecting for cells which express high levels of the Factor IX protein product;
(c) transfecting the selected cells with one or more processing factor(s) operably linked to a promoter;
(d) repeating step (b);
(e) optionally, repeating steps (a) and/or (c) followed by (b);
(f) cloning the selected cells;
(g) amplifying the cloned cells; and
(h) harvesting the product from the cloned cells in an amount of at least 15 mg/L recombinant biologically active Factor IX protein.

Further aspects, features and advantages of this invention will become apparent from the detailed description of the preferred embodiments which follow.

### Brief Description of the Drawing

These and other feature of this invention will now be described with reference to the drawings of preferred embodiments which are intended to illustrate and not to limit the invention.

The Figure shows the total amount of Factor IX produced per clone after transfection of a wild-type Factor IX gene into CHO cells. The Factor IX gene was under the control of the CHEF-1 promotor. Cells were allowed to grow in 5% serum for 14 days. The cell culture medium was harvested and the total amount of Factor IX antigen in µg per mL was quantified by a Factor IX ELISA method.

### DETAILED DESCRIPTION

Preferred embodiments of the invention are directed to methods for creating a genetically engineered cell that produces a high percentage of biologically active Factor IX protein in quantities suitable for commercialization world wide. Embodiments of the invention are described with respect to production of Factor IX. However, the disclosed methods are applicable to all vitamin K dependent proteins.

To produce low cost vitamin K-dependent protein biotherapeutics for commercial use on a worldwide basis, a genetically engineered cell must be created for production that (1) produces large quantities of the polypeptide chain that has the desired primary structure and (2) is capable of efficiently performing all' of the essential post-translational modifications that are needed to produce a fully functional synthetic biophaimaceutical product.

As used herein, the term "commercial use" means a Factor IX or other vitamin K dependent protein which, when produced from tissue culture cells, is at least 10% biologically active and is capable of production at a level of at least about 30 mg/L.

As used herein, "biologically activity" is determined with reference to a Factor IX standard derived from human plasma, such as MONONINE® (ZLB Behring). The biological activity of the Factor IX standard is taken to be 100%. Preferably, the Factor IX according to embodiments of the invention has at least 20% of the activity of the Factor IX standard, more preferably at least 25% of the activity of the Factor IX standard, more preferably at least 30% of the activity of the Factor IX standard, more preferably at least 35% of the activity of the Factor IX standard, more preferably at least 40% of the activity of the Factor IX standard, more preferably at least 45% of the activity of the Factor IX standard, more preferably at least 50% of the activity of the Factor IX standard, more preferably at least 55% of the activity of the Factor IX standard, more preferably at least 60% of the activity of the Factor IX standard, more preferably at least 65% of the activity of the Factor IX standard, more preferably at least 70% of the activity of the Factor IX standard, more preferably at least 75% of the activity of the Factor IX standard, more preferably at least 80% of the activity of the Factor IX standard, more preferably at least 85% of the activity of the Factor IX standard, more preferably at least 90% of the activity of the Factor IX standard.

Factor IX according to the invention is capable of production at a level of at least about 20 mg/L, preferably at least about 30 mg/L, more preferably at least about 40 mg/L, more preferably at least about 50 mg/L, yet more preferably at least about 60 mg/L, yet more preferably at least about 70 mg/L, yet more preferably at least about 80 mg/L, yet more preferably at least about 90 mg/L, yet more preferably at least about 100 mg/L, yet more preferably at least about 110 mg/L, yet more preferably at least about 120 ml/L, yet more preferably at least about 130 mg/L, yet more preferably at least about 140 mg/L, yet more preferably at least about 150 mg/L, yet more preferably at least about 160 mg/L, yet more preferably at least about 170 mg/L, yet more preferably at least about 180 mg/L, yet more preferably at least about 190 mg/L, yet more preferably at least about 200 mg/L, yet more preferably at least about 210 mg/L of biologically active vitamin K dependent protein.

The term "processing factor" is a broad term which includes any protein, peptide, non-peptide cofactor, substrate or nucleic acid which promotes the formation of a functional vitamin K dependent protein. Examples of such processing factors include, but are not limited to, PACE, VKOR and VKGC.

"Limit dilution cloning" has its usual and customary meaning and refers to a process of obtaining a monoclonal cell population starting from a polyclonal mass of cells. The starting (polyclonal) culture is serially diluted until a monoclonal culture is obtained.

Genetics Institute has shown that the production of large quantities of vitamin K dependent proteins is possible in genetically engineered CHO cells (U.S. Patent No. 4,770,999), but the percentage of fully functional protein is very low. An object of the present invention is a genetically engineered CHO cell that produces large quantities of vitamin K-dependent proteins whereby the percentage of fully functional protein is adequate to produce a low cost biopharmaceutical product for commercial use on a worldwide basis.

Stafford (U.S. Patent No. 5,268,275) has shown that the production of a high percentage of gamma-carboxylated vitamin K dependent proteins is possible in genetically engineered HEK 293 cells that are created to co-express enzymes that enhance the carboxylation of vitamin K-dependent proteins, but the total amount of gamma-carboxylated protein that is produced is very low. As dscribed herein is a genetically engineered HEK 293, CHO or other cell that produces large quantities of vitamin K-dependent proteins whereby the percentage of fully functional protein is adequate to produce a low cost biopharmaceutical product for commercial use on a worldwide basis.

Many transfection methods to create genetically engineered cells that express large quantities of recombinant proteins are well known. Monoclonal antibodies, for example, are routinely manufactured from genetically engineered cells that express protein levels in excess of 1000 mg/L. The present invention is not dependent on any specific transfection method that might be used to create a genetically engineered cell.

Many expression vectors can be used to create genetically engineered cells. Some expression vectors are designed to express large quantities of recombinant proteins after amplification of transfected cells under a variety of conditions that favor selected, high expressing, cells. Some expression vectors are designed to express large quantities of recombinant proteins without the need for amplification under selection pressure. The present invention is not dependent on the use of any specific expression vector.

To create a genetically engineered cell to produce large quantities of a given vitamin K-dependent protein, cells are transfected with an expression vector that contains the cDNA encoding the protein. The present invention requires that a transfected cell is created that is capable, under optimized growth conditions, of producing a minimum of 20 mg/L of the target vitamin K-dependent protein. Higher levels of production of the target vitamin K-dependent protein may be achieved and could be useful in the present invention. However, the optimum level of production of the target vitamin K-dependent protein is a level at or above 20 mg/L that can be obtained in a significantly increased functional form when the target protein is expressed with selected co-transfected enzymes that cause proper post-translational modification of the target protein to occur in a given cell system.

To create a genetically engineered cell that is capable of efficiently performing all of the essential post-translational modifications that are needed to produce a fully functional synthetic biopharmaceutical product, selected enzymes are co-transfected along with the vitamin K-dependent protein. Genetics Institute has shown that genetically engineered CHO cells that produce large quantities of vitamin K-dependent protein (Factor IX) have not been properly processed to remove the propeptide region prior to secretion. In this case, Genetics Institute has found that co-expression of an enzyme (PACE), known to remove the propeptide region from vitamin K-dependent proteins, substantially eliminates the deficiency in the intrinsic cellular levels of the enzyme. However, Genetics Institute has also shown that deficiencies in the intrinsic levels of other enzymes result in the majority of the vitamin K-dependent protein produced by genetically engineered CHO cells to be non-functional due to the low percentage of post-translational gamma-carboxylation of the gla-domain (Bond, M., Jankowski, M., Patel, H., Karnik, S., Strand, A., Xu, B., et al. [1998] Biochemical characterization of recombinant factor IX. Semin. Hematol. 35 [2 Suppl.2], 11-17).

The method of the present invention involves the first selection of a cell that may be genetically engineered to produce large quantities of a vitamin K-dependent protein such as Factor IX.

The cell may be selected from a variety of sources, but is otherwise a cell that may be transfected with an expression vector containing a nucleic acid, preferably a cDNA of a vitamin K-dependent protein.

From a pool of transfected cells, clones are selected that produce quantities of the Factor IX protein over a range (Target Range) that extends from the highest level to the lowest level that is minimally acceptable for the production of a commercial product. Cell clones that produce quantities of Factor IX protein within the Target Range may be combined to obtain a single pool or multiple sub-pools that divide the clones into populations of clones that produce high, medium or low levels of the vitamin K-dependent protein within the Target Range.

It is considered to be within the scope of the present invention that transfected cells that produce a Factor IX protein within the Target Range may be analyzed to determine the extent to which fully functional protein is produced. Such analysis will provide insight into the specific enzyme deficiencies that limit the production of fully functional protein. Further, it is anticipated that analysis of sub-pools consisting of cell clones that produce high, medium, or low levels of the vitamin K-dependent protein within the Target Range will provide insight into the specific enzyme deficiencies that limit the production of fully functional protein at varying levels of production of the vitamin K-dependent protein. Such analysis, whether done on a single pool of cell clones or on sub-pools, might reveal the specific enzyme deficiencies that must be eliminated to produce fully functional protein.

To eliminate the enzyme deficiencies within a pool of transfected clones that limits the production of fully functional vitamin K-dependent protein within the Target Range, embodiments of the present invention provide for the transfection of the pool of cells with an expression vector containing a nucleic acid, preferably a cDNA for a protein that, when expressed by a cell clone, will mitigate the enzyme deficiency in whole or in part. In preferred embodiments, it is further contemplated that more than one enzyme deficiency may be mitigated or that mitigation of a deficiency in post-translational modification of the vitamin K-dependent protein requires the presence of the activities of more than one enzyme or protein or other processing factor that may be provided in the method of the present invention by the simultaneous or subsequent (sequential) transfection of the cell clones with additional expression vectors containing cDNA for given proteins.

The host cell may first be transfected with gene(s) encoding one or more processing factors and subsequently transfected with a gene encoding a Factor IX protein. In some embodiments, the host cell is first transfected with a gene encoding a Factor IX protein and subsequently transfected with one or more processing factors. Optionally, the host cell may be transfected with the gene(s) for the processing factor(s) or with the gene for the Factor IX protein that is the same or substantially the same as an earlier transgene. After each round of transfection, clones are selected which express optimal levels of the transgene.

One such protein would have the enzymatic activity of vitamin K epoxide reductase (VKOR). Another such enzyme would have the enzymatic activity of vitamin K-dependent gamma-glutamyl carboxylase (VKGC).

Described herein is a method to identify the minimum protein transfection requirements to obtain a high percentage of fully functional Factor IX protein from a cell clone that produces the Factor IX protein in a quantity within the Target Range.

In preferred embodiments of the present invention, pools of cell clones that produce a Factor IX protein within the Target Range are subsequently transfected to provide a specific protein or multiple proteins in various combinations. Transfected pools of cell clones are then analyzed to determine the relative percentages of fully functional Factor IX protein that are now produced by transfectant pools that co-express the various proteins. The transfectant pool that produces the highest percentage of fully functional Factor IX protein with the minimum number of co-expressed proteins, is selected for subsequent cloning.

In preferred embodiments of the present invention, the selected transfectant pool is cloned to determine the optimal level of production of fully functional Factor IX protein that is attained by co-expression of additional protein(s). It is contemplated that higher percentages of fully functional Factor IX protein will be produced by cell clones that produce lower total amounts of the Factor IX protein within the Target Range. In some embodiments, some cell clones may be superproducers of Factor IX protein without significant improvements in post translational processing. Nevertheless, such superproducer lines produce usable amounts of functional protein as the overall production level is high. In preferred embodiments, the optimal level of production will be the highest level of functional Factor IX protein.

The practice of the present invention employs, unless otherwise indicated, conventional techniques of molecular biology, microbiology, recombinant DNA, and immunology, which are within the skill of the art. Such techniques are explained fully in the literature. See, e.g., Sambrook, et al., "Molecular Cloning; A Laboratory Manual", 2nd ed (1989); "DNA Cloning", Vols. I and II (D. N Glover ed. 1985); "Oligonucleotide Synthesis" (M. J. Gait ed. 1984); "Nucleic Acid Hybridization" (B. D. Hames & S. J. Higgins eds. 1984); "Transcription and Translation" (B. D. Hames & S. J. Higgins eds. 1984); "Animal Cell Culture" (R. I. Freshney ed. 1986); "Immobilized Cells and Enzymes" (IRL Press, 1986); B. Perbal, "A Practical Guide to Molecular Cloning" (1984); the series, Methods in Enzymology (Academic Press, Inc.), particularly Vols. 154 and 155 (Wu and Grossman, and Wu, eds., respectively); "Gene Transfer Vectors for Mammalian Cells" (J. H. Miller and M. P. Calos eds. 1987,Cold Spring Harbor Laboratory);"Immunochemical Methods in Cell and Molecular Biology", Mayer and Walker, eds. (Academic Press, London, 1987); Scopes, "Protein Purification: Principles and Practice", 2nd ed. 1987 (Springer-Verlag, N.Y.); and "Handbook of Experimental Immunology" Vols I-IV (D. M. Weir and C. C. Blackwell eds 1986).

### Modification of the propeptide

In some embodiments, γ-carboxylation is increased by replacing the native propeptide sequence with a propeptide sequence that has a lower affinity for the gamma carboxylase as discussed in U.S. Application No. 2003/0220247. Useful propeptide sequences include altered forms of wild type sequences or propeptide sequences, or combinations of the same, for heterologous vitamin K dependent proteins. The propeptide sequence in vitamin K-dependent proteins is the recognition element for the enzyme which directs gamma carboxylation of the protein. Vitamin K-dependent proteins are not fully functional unless they comprise a high percentage of gamma carboxylated moieties. Thus, it is important when generating recombinant versions of these proteins that mechanisms be put in place to ensure full gamma carboxylation of the same.

The sequence alignment of several propeptide sequences is shown in FIG. 3 of US. 2003/0220247. Thus, propeptides which are useful in the present invention are those which have the sequences shown in FIG. 3 wherein an 18 amino acid sequence of several useful propeptides is shown along with the relative affinities of these propeptides for gamma carboxylase. A low affinity propeptide may be generated by modifying any one of amino acids -9 or -13 on either prothrombin or protein C. Preferred modifications include the substitution of an Arg or a His residue at position -9 and the substitution of a Pro or a Ser residue at position -13. Other preferred chimeric proteins include a propeptide selected from the group consisting of altered Factor IX, or an unaltered propeptide in combination with the mature Factor IX protein which is not native to the chosen propeptide sequence.

The term "fully gamma carboxylated protein" is used herein to refer to a protein wherein at least about 80% of the amino acids which should be gamma carboxylated are carboxylated. Preferably, at least about 85%, more preferably, at least about 90%, more preferably at least about 95% and even more preferably, at least about 99% of the amino acids which should be gamma carboxylated are gamma carboxylated.

### Paired basic amino acid converting enzyme (PACE)

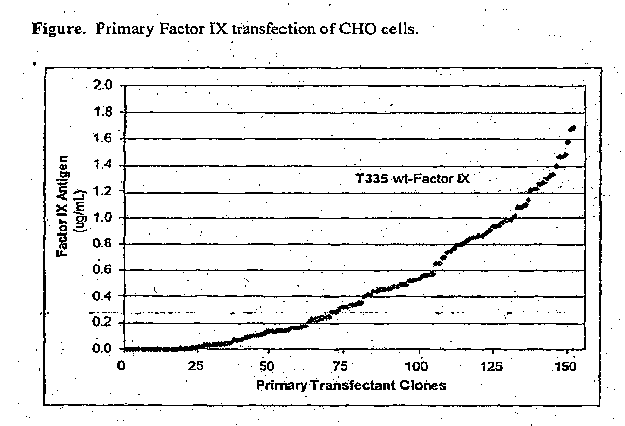

As used herein, the term "PACE" is an acronym for paired basic amino acid converting (or cleaving) enzyme. PACE, originally isolated from a human liver cell line, is a subtilisin-like endopeptidase, i.e., a propeptide-cleaving enzyme which exhibits specificity for cleavage at basic residues of a polypeptide, e.g., -Lys-Arg-, -Arg-Arg, or -Lys-Lys-. PACE is stimulated by calcium ions; and inhibited by phenylmethyl sulfonyl fluoride (PMSF). A DNA sequence encoding PACE (or furin) appears in FIG. 1 [SEQ ID NO: 1] of U.S. Patent No. 5,460,950, which is incorporated herein by reference. The co-expression of PACE and a proprotein which requires processing for production of the mature protein results in high level expression of the mature protein. Additionally, co-expression of PACE with proteins requiring γ-carboxylation for biological activity permits the expression of increased yields of functional, biologically active mature proteins in eukaryotic, preferably mammalian, cells.

### Vitamin K dependent epoxide reductase

Vitamin K dependent epoxide reductase (VKOR) is important for vitamin K dependent proteins because vitamin K is converted to vitamin K epoxide during reactions in which it is a cofactor. The amount of vitamin K in the human diet is limited. Therefore, vitamin K epoxide must be converted back to vitamin K by VKOR to prevent depletion. Consequently, co-transfection with VKOR provides sufficient vitamin K for proper functioning of the vitamin K dependent enzymes such as the vitamin K dependent γ-glutamyl carboxylase (VKCG). Proper functioning of vitamin K dependent VKCG is essential for proper γ-carboxylation of the gla-domain of vitamin K dependent coagulation factors.

### Vitamin K dependent gamma carboxylase

Vitamin K dependent γ-glutamyl carboxylase (VKGC) is an ER enzyme involved in the post-translation modification of vitamin K dependent proteins. VKGC incorporates CO₂ into glutamic acid to modify multiple residues within the vitamin K dependent protein within about 40 residues of the propeptide. The loss of three carboxylations markedly decreases the activity of vitamin K-dependent proteins such as vitamin K dependent coagulation factors. The cDNA sequence for human vitamin K dependent γ-glutamyl carboxylase is described by U.S. Patent No. 5,268,275. The sequence is provided in SEQ ID NO: 15 of U.S. Patent No. 5,268,275.

### Genetic Engineering Techniques

The production of cloned genes, recombinant DNA, vectors, transformed host cells, proteins and protein fragments by genetic engineering is well known. See, e.g., U.S. Pat. No. 4,761,371 to Bell et al. at Col. 6 line 3 to Col. 9 line 65; U.S. Pat. No. 4,877,729 to Clark et al. at Col. 4 line 38 to Col. 7 line 6; U.S. Pat. No. 4,912,038 to Schilling at Col. 3 line 26 to Col. 14 line 12; and U.S. Pat. No. 4,879,224 to Wallner at Col. 6 line 8 to Col. 8 line 59.

A vector is a replicable DNA construct. Vectors are used herein either to amplify DNA encoding Factor IX and/or to express DNA which encodes Factor IX. An expression vector is a replicable DNA construct in which a DNA sequence encoding Factor IX is operably linked to suitable control sequences capable of effecting the expression of Factor IX protein in a suitable host. The need for such control sequences will vary depending upon the host selected and the transformation method chosen. Generally, control sequences include a transcriptional promoter, an optional operator sequence to control transcription, a sequence encoding suitable mRNA ribosomal binding sites, and sequences which control the termination of transcription and translation.

Amplification vectors do not require expression control domains. All that is needed is the ability to replicate in a host, usually conferred by an origin of replication, and a selection gene to facilitate recognition of transformants.

Vectors comprise plasmids, viruses (e.g., adenovirus, cytomegalovirus), phage, and integratable DNA fragments (i.e., fragments integratable into the host genome by recombination). The vector replicates and functions independently of the host genome, or may, in some instances, integrate into the genome itself. Expression vectors should contain a promoter and RNA binding sites which are operably linked to the gene to be expressed and are operable in the host organism.

DNA regions are operably linked or operably associated when they are functionally related to each other. For example, a promoter is operably linked to a coding sequence if it controls the transcription of the sequence; or a ribosome binding site is operably linked to a coding sequence if it is positioned so as to permit translation.

Transformed host cells are cells which have been transformed or transfected with one or more Vitamin K dependent protein vector(s) constructed using recombinant DNA techniques.

### Expression of multiple proteins

Embodiments of the invention are directed to providing the cell with the necessary enzymes and cofactors to process Factor IX proteins so that higher yields of biologically active proteins are achieved. When adequate levels of fully functional proteins are produced by a recombinant cell, lengthy purification steps designed to remove the useless, partially modified, or unmodified protein from the desired product are avoided. This lowers the production cost and eliminates inactive material that may have undesirable side effects for the patient.

In preferred embodiments, methods for producing Factor IX proteins by co-expression with VKGC and VKOR can include the following techniques. First, a single vector containing coding sequences for more than one protein and a Factor IX protein can be inserted into a selected host cell. PACE and a Factor IX protein can be inserted into a selected host cell. Alternatively, two or more separate vectors encoding a Factor IX protein plus one or more other proteins, can be inserted into a host. Upon culturing under suitable conditions for the selected host cell, the two or more polypeptides are produced and interact to provide cleavage and modification of the proprotein into the mature protein.

Another alternative is the use of two transformed host cells wherein one host cell expresses the Factor IX protein and the other host cell expresses one or more of VKGC and VKOR which will be secreted into the medium. These host cells can be co-cultured under conditions which allow expression and secretion or release of the recombinant Factor IX protein and the co-expressed recombinant polypeptides, gamma carboxylation of N-terminal glutamates.

In some instances, it may be desirable to have a plurality of copies, two or more, of the gene expressing the Factor IX protein in relation to the other genes, or vice versa. This can be achieved in a variety of ways. For example, one may use separate vectors or plasmids, where the vector containing the Factor IX protein encoding polynucleotide has a higher copy number than the vector containing the other polynucleotide sequences, or vice versa. In this situation, it would be desirable to have different selectable markers on the two plasmids, so as to ensure the continued maintenance of the plasmids in the host. Alternatively, one or both genes could be integrated into the host genome, and one of the genes could be associated with an amplifying gene, (e.g., dhfr or one of the metallothionein genes).

Alternatively, one could employ two transcriptional regulatory regions having different rates of transcriptional initiation, providing for the enhanced expression of either Factor IX protein or the expression of any of the other processing factor polypeptides, relative to Factor IX protein. As another alternative, one can use different promoters, where one promoter provides for a low level of constitutive expression of Factor IX protein, while the second promoter provides for a high level of induced expression of the other products. A wide variety of promoters are known for the selected host cells, and can be readily selected and employed in the invention by one of skill in the art such as CMV, MMTV, SV 40 or SRα promoters which are well known mammalian promoters.

In a preferred embodiment, a promoter for the elongation factor -1α from Chinese hamster is used (CHEF1) to provide high level expression of a Factor IX coagulation factor and/or processing factor(s). The CHEF1 vector is used as described in Deer, et al.(2004) "High-level expression of proteins in mammalian cells using transcription regulatory sequences from the Chinese Hamster EF-1α gene" Biotechnol. Prog. 20: 880-889 and in U.S. Patent No. 5,888,809. The CHEF1 vector utilizes the 5' and 3' flanking sequences from the Chinese hamster EF-1α. The CHEF1 promoter sequence includes approximately 3.7 kb DNA extending from a SpeI restriction site to the initiating methionine (ATG) codon of the EF-1α protein. The DNA sequence is set forth in SEQ ID NO: 1 of U.S. Patent No. 5,888,809.

Production of biologically active Factor IX, are maximized by overexpression of one or more of VKOR, and VKGC and/or by modification of the gla region to maximize γ-carboxylation. That is, rate limiting components are expressed in sufficient quantity so that the entire system operates to produce a commercially viable quantity of Factor IX protein.

### Host cells

Suitable host cells include prokaryote, yeast or higher eukaryotic cells such as mammalian cells and insect cells. Cells derived from multicellular organisms are a particularly suitable host for recombinant Vitamin K Dependent protein synthesis, and mammalian cells are particularly preferred. Propagation of such cells in cell culture has become a routine procedure (Tissue Culture, Academic Press, Kruse and Patterson, editors (1973)). Examples of useful host cell lines are VERO and HeLa cells, Chinese hamster ovary (CHO) cell lines, and WI138, HEK 293, BHK, COS-7, CV, and MDCK cell lines. Expression vectors for such cells ordinarily include (if necessary) an origin of replication, a promoter located upstream from the DNA encoding vitamin K dependent protein(s) to be expressed and operatively associated therewith, along with a ribosome binding site, an RNA splice site (if intron-containing genomic DNA is used), a polyadenylation site, and a transcriptional termination sequence. In a preferred embodiment, expression is carried out in Chinese Hamster Ovary (CHO) cells using the expression system of U.S. Patent No. 5,888,809.

The transcriptional and translational control sequences in expression vectors to be used in transforming vertebrate cells are often provided by viral sources. For example, commonly used promoters are derived from polyoma, Adenovirus 2, and Simian Virus 40 (SV40). See. e.g.. U.S. Pat. No. 4,599,308.

An origin of replication may be provided either by construction of the vector to include an exogenous origin, such as may be derived from SV 40 or other viral (e.g. Polyoma, Adenovirus, VSV, or BPV) source, or may be provided by the host cell chromosomal replication mechanism. If the vector is integrated into the host cell chromosome, the latter is often sufficient.

Rather than using vectors which contain viral origins of replication, one can transform mammalian cells by the method of cotransformation with a selectable marker and the DNA for the Vitamin K Dependent protein(s). Examples of suitable selectable markers are dihydrofolate reductase (DHFR) or thymidine kinase. This method is further described in U.S. Pat. No. 4,399,216.

Other methods suitable for adaptation to the synthesis of Vitamin K Dependent protein(s) in recombinant vertebrate cell culture include those described in M-J. Gething et al., Nature 293, 620 (1981); N. Mantei et al., Nature 281, 40; A. Levinson et al., EPO Application Nos. 117,060A and 117,058A.

Host cells such as insect cells (e.g., cultured Spodoptera frugiperda cells) and expression vectors such as the baculovirus expression vector (e.g., vectors derived from Autographa californica MNPV, Trichoplusia ni MNPV, Rachiplusia ou MNPV, or Galleria ou MNPV) may be employed in carrying out the present invention, as described in U.S. Pat. Nos. 4,745,051 and 4,879,236 to Smith et al. In general, a baculovirus expression vector comprises a baculovirus genome containing the gene to be expressed inserted into the polyhedrin gene at a position ranging from the polyhedrin transcriptional start signal to the ATG start site and under the transcriptional control of a baculovirus polyhedrin promoter.

Prokaryote host cells include gram negative or gram positive organisms, for example Escherichia coli (E. coli) or Bacilli. Higher eukaryotic cells include established cell lines of mammalian origin as described below. Exemplary host cells are E. coli W3110 (ATCC 27,325), E. coli B, E. coli X1776 (ATCC 31,537), E. coli 294 (ATCC 31,446). A broad variety of suitable prokaryotic and microbial vectors are available. E. coli is typically transformed using pBR322. Promoters most commonly used in recombinant microbial expression vectors include the betalactamase (penicillinase) and lactose promoter systems (Chang et al., Nature 275, 615 (1978); and Goeddel et al., Nature 281, 544 (1979)), a tryptophan (trp) promoter system (Goeddel et al., Nucleic Acids Res. 8, 4057 (1980) and EPO App. Publ. No. 36,776) and the tac promoter (H. De Boer et al., Proc. Natl. Acad. Sci. USA 80, 21 (1983)). The promoter and Shine-Dalgarno sequence (for prokaryotic host expression) are operably linked to the DNA encoding the Vitamin K Dependent protein(s), i.e., they are positioned so as to promote transcription of Vitamin K Dependent Protein(s) messenger RNA from the DNA.

Eukaryotic microbes such as yeast cultures may also be transformed with Vitamin K Dependent Protein-encoding vectors. see, e.g., U.S. Pat. No. 4,745,057. Saccharomyces cerevisiae is the most commonly used among lower eukaryotic host microorganism, although a number of other strains are commonly available. Yeast vectors may contain an origin of replication from the 2 micron yeast plasmid or an autonomously replicating sequence (ARS), a promoter, DNA encoding one or more Vitamin K Dependent proteins, sequences for polyadenylation and transcription termination, and a selection gene. An exemplary plasmid is YRp7, (Stinchcomb et al., Nature 282, 39 (1979); Kingsman et al., Gene 7, 141 (1979); Tschemper et al., Gene 10, 157 (1980)). Suitable promoting sequences in yeast vectors include the promoters for metallothionein, 3-phosphoglycerate kinase (Hitzeman et al., J. Biol. Chem. 255, 2073 (1980) or other glycolytic enzymes (Hess et al., J. Adv. Enzyme Reg. 7,149 (1968); and Holland et al., Biochemistry 17, 4900 (1978)). Suitable vectors and promoters for use in yeast expression are further described in R Hitzeman et al., EPO Publn. No. 73,657.

Cloned genes employed in the present invention may code for any species of origin, including mouse, rat, rabbit, cat, porcine, and human, but preferably code for Factor IX proteins of human origin. DNA encoding Factor IX proteins that is' hybridizable with DNA encoding for proteins disclosed herein is also encompassed. Hybridization of such sequences may be carried out under conditions of reduced stringency or even stringent conditions (e.g., conditions represented by a wash stringency of 0.3M NaCl, 0.03M sodium citrate, 0.1% SDS at 60° C or even 70° C to DNA encoding the vitamin K dependent protein disclosed herein in a standard in situ hybridization assay. See J. Sambrook et al., Molecular Cloning, A Laboratory Manual (2d Ed. 1989)(Cold Spring Harbor Laboratory)).

As noted above, preferred embodiments of the present invention provide methods of providing functional Vitamin K dependent proteins by methods which include carboxylation of the N-terminal glu residues. The strategy may include co-expressing Vitamin K dependent protein along with VKOR and VKGC in a single host cell. In general, the method comprises culturing a host cell which expresses a vitamin K dependent protein and supporting proteins; and then harvesting the proteins from the culture. While some host cells may provide some Vitamin K dependent protein, VKOR, and VKGC at basal levels, in preferred embodiments, the vector DNA encoding VKGC and VKOR is included to enhance carboxylation. The culture can be carried out in any suitable fermentation vessel, with a growth media and under conditions appropriate for the expression of the vitamin K dependent protein(s) by the particular host cell chosen. The Vitamin K dependent protein harvested from the culture is found to be carboxylated due to the expression of the supporting proteins in the host cell. In preferred embodiments, vitamin K dependent protein can be collected directly from the culture media, or the host cells lysed and the vitamin K dependent protein collected therefrom. In preferred embodiments, vitamin K dependent protein can then be further purified in accordance with known techniques.

As a general proposition, the purity of the recombinant protein produced according to the present invention will preferably be an appropriate purity known to the skilled art worker to lead to the optimal activity and stability of the protein. For example, when the recombinant protein is Factor IX , the Factor IX is preferably of ultrahigh purity. Preferably, the recombinant protein has been subjected to multiple chromatographic purification steps, such as affinity chromatography, ion-exchange chromatography and preferably immunoaffinity chromatography to remove substances which cause fragmentation, activation and/or degradation of the recombinant protein during manufacture, storage and/or use. Illustrative examples of such substances that are preferably removed by purification include thrombin and Factor IXa; other protein contaminants, such as modification enzymes like PACE/furin, VKOR, and VKGC; proteins, such as hamster proteins, which are released into the tissue culture media from the production cells during recombinant protein production; non-protein contaminants, such as lipids; and mixtures of protein and non-protein contaminants, such as lipoproteins. Purification procedures for vitamin K dependent proteins are known in the art. For example, see U.S. Patent No, 5,714,583.

Factor IX DNA coding sequences, along with vectors and host cells for the expression thereof, are disclosed in European Patent App. 373012, European Patent App. 251874, PCT Patent Appl. 8505376, PCT Patent Appln. 8505125, European Patent Appln. 162782, and PCT Patent Appln. 8400560. Genes for other coagulation factors are also known and available, for example, Factor II (Accession No. NM_000506), Factor VII (Accession No. NM_019616, and Factor X (Accession No. NM_000504).

### EXAMPLES

### Example 1. Primary transfection of CHO cells with Factor IX gene.

A wild-type. Factor IX gene was transfected into CHO cells by limit dilution into 96-well plates. The Factor IX gene was under the control of the CHEF-1 promotor. Cells were allowed to grow in 5% serum for 14 days. The cell culture medium was harvested and the total amount of Factor IX antigen in µg per mL was quantified by a Factor IX ELISA method. More than 150 clones were evaluated and the total amount of Factor IX produced per clone is reported in Figure 1.

CHO cells transfected with the Factor IX gene produced Factor IX antigen which was detected by Factor IX ELISA. The amount varied significantly between clones. The range of total protein production after 14 days in culture was between 0 and greater than 1.6 µg/mL of culture medium. Although not determined in this experiment the Factor IX produced in primary transfectants was about 20% biologically active (data not shown) as determined in an APTT clotting assay using Factor IX-deficient plasma. Factor IX antigen can therefore be produced in CHO cells following transfection of the cells with wild type Factor IX.

### Example 2. Supertransfection of Factor IX-producing CHO cells with VKGC and VKOR genes.

In order to increase the percentage of active Factor IX produced in Factor IX-transfected CHO cells, the primary transfectants were pooled, expanded in tissue culture. and supertransfected with vectors containing cDNA for enzymes generally thought to be important for the efficient Vitamin K-dependent gamma-carboxylation of Factor IX. Factor IX producing clones were pooled in a shake flask and supertransfected with cDNAs for both Vitamin K-dependent gamma-carboxylase (VKGC) and Vitamin K-dependent epoxide reductase (VKOR). Individually supertransfected cells were grown by limit dilution in 96-well plates in 5% serum for 14 days. The total amount of Factor IX antigen produced per mL was measured by Factor IX ELISA. The amount of active Factor IX was measured by an APTT clotting assay using Factor IX-deficient plasma as substrate and plasma-derived Factor IX as standard.

**Table 1. Supertransfection of Factor IX-producing CHO cell clones with VKGC and VKOR**

| **Clone** | **Titer (µg/mL)** | **FIX Activity (U/mL)** | **Specific Activity (U/mg)** | **Active FIX (µg/mL)** | **% Active FIX** |
|---|---|---|---|---|---|
| **1** | **1.560** | **0.15** | **97** | **0.549** | **35** |
| **2** | **1.283** | **0.10** | **76** | **0.356** | **28** |
| **3** | **0.469** | **0.09** | **198** | **0.338** | **72** |
| **4** | **1.628** | **0.09** | **56** | **0.331** | **20** |
| **5** | **2.205** | **0.09** | **41** | **0.331** | **15** |
| **6** | **0.604** | **0.09** | **144** | **0.316** | **52** |
| **7** | **1.274** | **0.09** | **68** | **0.316** | **25** |
| **8** | **0.811** | **0.09** | **105** | **0.309** | **38** |
| **9** | **0.827** | **0.08** | **100** | **0.302** | **36** |
| **10** | **0.954** | **0.07** | **77** | **0.265** | **28** |
| **11** | **0.177** | **0.03** | **186** | **0.120** | **68** |
| **12** | **0.340** | **0.06** | **171** | **0.211** | **62** |
| **13** | **0.121** | **0.02** | **165** | **0.073** | **60** |
| **14** | **0.272** | **0.04** | **143** | **0.142** | **52** |
| **15** | **0.169** | **0.02** | **142** | **0.087** | **52** |

As seen in **Table 1**, the results of 15 individual clones were analyzed. The Factor IX antigen varied between 0.12 and 2.2 µg/mL. The percentage of active Factor IX ranged between 15 and 72%. Consequently, the supertransfection of Factor IX producing cells with VKGC and VKOR significantly increases the percentage of active Factor IX being produced by specific CHO cell clones.

Note more antigen is produced as production is scaled up. For example, for 6-well plates, about 25-fold more antigen is produced when compared to 96-well plates. Consequently, in 6-well plates the levels of Factor IX antigen would be expected to range from 3-55 µg/ml.

### Example 3. Large-scale production of large quantities of biologically active recombinant Factor IX

To demonstrate that Factor IX-producing CHO cells supertransfected with VKGC and VKOR can produce large quantities of biologically active Factor IX, two independently isolated clones were grown in bioreactors and the quantity and quality of Factor IX product were evaluated after purifying the material. Bioreactors containing serum free medium were used to grow Clone 130 (12 L bioreactor) and Clone 44 (10 L bioreactor). Both of these clones expressed human Factor IX, VKGC and VKOR. The bioreactors were allowed to grow for 12 days without media change. The tissue culture fluid was separated from the cells and the Factor IX purified by a standard set of chromatography columns, resulting in Factor IX protein with greater than 90% purity.

**Table 2. Large-Scale Production of biologically active recombinant Factor IX**

| **Clone Grown in Bioreactor** | **Total Titer (mg/L)** | **% Active** | **Active Titer (mg/L)** |
|---|---|---|---|
| 130 | 44 | 61 | 27 |
| 44 | 28 | 35 | 10 |

As presented in Table 2, large quantities of Factor IX antigen were produced in both bioreactors. Clone 130 produced 44 mg of Factor IX per L of culture medium and Clone 44 produced 28 mg of Factor IX per L. Consistent with data presented earlier, the % active Factor IX was seen to be between 35 and 61 %. Consequently, Factor IX producing CHO cells, when supertransfected with the posttranslational modification enzymes VKGC and VKOR, produce large quantities of Factor IX antigen that contains a significant amount of biologically active Factor IX.

### Example 4. Re-transfection with VKOR of clones producing Factor IX, VKGC and VKOR

In order to determine if it is possible to produce biologically active recombinant Factor IX in transfected CHO cells, the two clones, 130 and 44; which produced Factor IX after being supertransfected with VKGC and VKOR, were re-transfected with VKOR. Individual isolates of Clones 130 and 44 were cloned by limit dilution and re-transfected with the cDNA for VKOR. The clones were grown up in 6-well plates and the cells were allowed to grow for 9 days until they were confluent. The total Factor IX antigen (µg per mL) was measured by Factor IX ELISA, and the activity (U per mL) was determined by an APTT clotting assay using Factor IX-deficient plasma.

**Table 3. Re-transfection with VKOR of CHO clones producing Factor IX, VKGC and VKOR.**

| **Clone** | **F-IX Titer(Ug/mL)** | **F-IX Activity (U/mL)** | **Specific Acitivity (U/mg)** | **% Active** |
|---|---|---|---|---|
| **130-1** | **2.7** | **0.55** | **199** | **80%** |
| **130-2** | **2.6** | **0.48** | **186** | **74%** |
| **130-3** | **2.6** | **0.55** | **209** | **84%** |
| **130-4** | **1.3** | **0.23** | **172** | **69%** |
| **130-5** | **1.7** | **0.38** | **229** | **91%** |
| **130-6** | **1.1** | **0.16** | **145** | **58%** |
| **130-7** | **1.7** | **0.29** | **172** | **69%** |
| **130-8** | **2.0** | **0.46** | **229** | **92%** |

| **Clone** | **F-IX Titer (Ug/mL)** | **F-IX Activity (U/mL)** | **Specific Acitivity (U/mg)** | **% Active** |
|---|---|---|---|---|
| **130-9** | **2.4** | **0.49** | **206** | **82%** |
| **130-10** | **1.9** | **0.42** | **222** | **89%** |
| **130-11** | **1.9** | **0.40** | **212** | **85%** |
| **130-12** | **2.1** | **0.50** | **237** | **95%** |
| **130-13** | **2.2** | **0.48** | **223** | **89%** |
| **130-14** | **2.4** | **0.63** | **265** | **106%** |
| **130-15** | **2.2** | **0.44** | **196** | **78%** |
| **130-16** | **1.4** | **0.31** | **214** | **86%** |
| **130-17** | **1.8** | **0.34** | **185** | **74%** |
| **130-18** | **1.5** | **0.27** | **176** | **70%** |
| **44-1** | **3.0** | **0.45** | **147** | **59%** |
| **44-2** | **0.9** | **0.22** | **235** | **94%** |
| **44-3** | **2.2** | **0.21** | **92** | **37%** |
| **44-4** | **1.3** | **0.26** | **210** | **84%** |
| **44-5** | **1.6** | **0.36** | **230** | **92%** |
| **44-6** | **1.1** | **0.22** | **194** | **78%** |
| **44-7** | **1.4** | **0.23** | **165** | **66%** |
| **44-8** | **0.9** | **0.15** | **163** | **65%** |
| **44-9** | **1.7** | **0.33** | **197** | **79%** |
| **44-10** | **1.6** | **0.25** | **156** | **62%** |
| **44-11** | **2.3** | **0.45** | **199** | **80%** |
| **44-12** | **1.4** | **0.34** | **240** | **96%** |
| **44-13** | **1.6** | **0.21** | **132** | **53%** |
| **44-14** | **1.9** | **0.26** | **136** | **55%** |
| **44-15** | **1.8** | **0.45** | **250** | **100%** |
| **44-16** | **2.1** | **0.42** | **194** | **77%** |

The results in Table 3 show that subclones of both Clone 130 and Clone 44 produced significant quantities of Factor IX antigen, ranging from 0.9 to 3.0 µg/mL. Furthermore, as a consequence of the re-transfection with VKOR, both clones yielded at least one subclone that produced 100% of the Factor IX as biologically active protein, as well as several subclones with greater than 90% active Factor IX. These data suggest that adequate co-expression of VKGC and VKOR can facilitate production of totally highly active or even totally active Factor IX in CHO cells transfected with a wild type Factor IX cDNA.

### Example 5. Large-scale production of biologically active Factor IX in genetically engineered cells re-transfected with the post-translational modification enzyme VKOR.

This experiment was designed to demonstrate that CHO cells producing recombinant Factor IX after transfection with VKGC and VKOR and re-transfected with VKOR can produce large quantities of Factor IX at production scale. Individual isolates of Clone 130 re-transfected with VKOR were grown up in 1.5 L shake flasks (to represent commercial production) and the Factor IX antigen and biological activity were measured. Individual subclones of clone 130 described in EXAMPLE 4 above (CHO clone transfected with Factor IX, VKGC and VKOR and subsequently re-transfected with VKOR) were isolated by limit dilution in 6-well microtiter plates and then seeded into 1.5 L shaker flasks. Production of Factor IX in 1.5 L shaker flasks is known to reflect production conditions of 15 L and larger bioreactors (data not shown). The cells were allowed to grow in serum free media for 18 days, at which point samples were taken and evaluated for Factor IX antigen by a Factor IX ELISA and for biological activity by APTT clotting assay using Factor IX-deficient plasma.

**Table 4. Production of large quantities of active Factor IX in CHO cells transfected with Factor IX, VKGC and VKOR, and re-transfected with VKOR**

| **Clone** | **Flask** | **Total Titer (mg/L)** | **% Active** | **Active Titer (mg/L)** |
|---|---|---|---|---|
| **130** | A | 42.0 | 46.0 | 19.3 |
| | B | 41.8 | 46.8 | 19.6 |
| | **Average** | **41.9 ± 0.1** | **46.4 ± 0.4** | **19.4 ± 0.1** |
| **130-6** | A | 45.1 | 49.5 | 22.3 |
| | B | 42.8 | 52.2 | 22.4 |
| | **Average** | **43.9±1.1** | **50.9±1.3** | **22.3 ±0.1** |
| **130-16** | A | 41.5 | 48.7. | 20.2 |
| | B | 37.1 | 51.3 | 19.0 |
| | **Average** | **39.3 ± 2.2** | **50.0 ± 1.3** | **19.6 ± 0.6** |
| **130-17** | A | 52.0 | 57.9 | 30.1 |
| | B | 45.0 | 70.8 | 31.9 |
| | **Average** | **48.5 ± 3.5** | **64.3 ± 6.4** | **31.0 ± 0.9** |
| **130-19** | A | 53.8 | 52.6 | 28.3 |
| | B | 50.6 | 59.0 | 29.8 |
| | **Average** | **52.2 ± 1.6** | **55.8 ± 3.2** | **29.1 ± 0.8** |
| **130-31** | A | 45.7 | 47.9 | 21.9 |
| | B | 44.1 | 49.3 | 21.7 |
| | **Average** | **44.9 ± 0.8** | **48.6 ± 10.7** | **21.8 ± 0.1** |

The data for Clone 130 itself and for five subclones are presented in Table 4. Large quantities of Factor IX antigen were produced by all clones, ranging from 39.3 to 52.2 mg of Factor IX antigen per Liter of culture fluid. The percentage of active Factor IX was also quite high, ranging from 46.4% to 64.3%. The amount of biologically active Factor IX produced was also surprisingly high ranging from 19.4 to 31.0 mg/L. Consequently, in shaker flask systems, which reflect the production of Factor IX in commercial level bioreactors, large quantities of Factor IX antigen and active Factor IX can be produced in cells that have be transfected with Factor IX, VKGC, VKOR and subsequently re-transfected with VKOR.

## Claims

1. A method of producing a recombinant biologically active Factor IX protein product, comprising the steps of:
transfecting a CHO cell, with a gene encoding the Factor IX protein product operably linked to a Chinese Hamster elongation factor 1-α (CHEF-1) promoter, and at least two genes operably linked to at least one promoter, either simultaneously or sequentially; and harvesting the Factor IX protein product,
whereby the cell produces biologically active Factor IX protein product, as measured with reference to the Factor IX standard Mononine, in an amount of at least 15 mg/L; and wherein the at least two genes comprise a gene encoding vitamin K dependent epoxide reductase (VKOR), and a gene encoding vitamin K dependent γ-glutamyl carboxylase (VKGC).

2. The method of claim 1, wherein at least one of the genes is overexpressed.

3. The method of claim 2, wherein the overexpressed gene is operably linked to a Chinese hamster elongation factor 1-a (CHEF1) promoter.

4. The method of claim 1, wherein at least about 75% of the glutamic acid residues within the gla-domain of the biologically active Factor IX protein product are gamma carboxylated.

5. The method of any proceeding claim, wherein at least 50%, at least 70% or at least 80% of the Factor IX protein is biologically active.

6. The method of any preceding claim, wherein the biologically active Factor IX protein is produced in an amount of at least 20 mg/L.

7. The method of any preceding claim, wherein transfection is sequential and wherein transfecting the mammalian cell further comprises: selecting for cells which express high levels of the Factor IX protein product, VKOR or VKGC; cloning the selected cells; and amplifying the cloned cells.

8. The method of claim 7, wherein the step of transfecting with the at least two genes is performed before the step of transfecting with the gene encoding the Factor IX protein.

9. The method of claim 7, wherein the step of transfecting with the gene encoding the Factor IX protein is performed before the step of transfecting with the at least two genes.

10. The method of any of claim 7 to 9, wherein the CHO cell is selected for expression of endogenous levels of one or more processing factors before transfection.

11. A recombinant CHO cell, comprising a gene encoding a Factor IX protein operably linked to a Chinese Hamster elongation factor I-α (CHEF-1) promoter and at least two genes operably linked to at least one promoter,
wherein the cell produces biologically active FIX protein in an amount of at least 15 mg/L, as measured with reference to the Factor IX standard Mononine,
wherein the at least two genes comprise a gene encoding vitamin K dependent epoxide reductase (VKOR) and a gene encoding vitamin K dependent γ-glutamyl carboxylase (VKGC).

12. The recombinant cell of claim 11, wherein at least one gene is over-expressed.

13. The recombinant cell of claim 12, wherein the overexpressed gene product is operably linked to a Chinese hamster elongation factor 1-α (CHEF 1) promoter.

14. A method of producing a recombinant biologically active Factor IX protein product, comprising the steps of: (a) transfecting a CHO cell, with a gene encoding the Factor IX protein operably linked to a Chinese hamster elongation factor 1-α (CHEF 1) promoter; (b) selecting for cells which express high levels of the Factor IX protein product; (c) transfecting the selected cells with at least two genes, wherein the at least two genes comprise a gene encoding vitamin K dependent epoxide reductase (VKOR), and a gene encoding vitamin K dependent γ-glutamyl carboxylase (VKGC); (d) repeating step (b); (e) optionally, repeating steps (a) and/or (c) followed by (b); (f) cloning the selected cells; (g) growing the cloned cells; and (h) harvesting the Factor IX protein product from the cloned cells in an amount of at least 15 mg/L.

## Patentansprüche

1. Verfahren zum Herstellen eines rekombinanten biologisch aktiven Faktor IX ProteinProdukts, welches folgende Schritte aufweist:
Transfizieren einer CHO-Zelle (CHO = Chinese Hamster Ovary = Zelle aus dem Ovar des chinesischen Hamsters) mit einem Gen, welches das Faktor IX-Proteinprodukt kodiert, welches funktionsfähig an einen Chinese Hamster Verlängerungs- bzw. Elongationsfaktor 1-α (CHEF-1 = Chinese Hamster elongation factor)-Promotor und mindestens zwei Genen, die funktionstüchtig an wenigstens einen Promotor gekoppelt sind, entweder gleichzeitig oder nacheinander, gekoppelt ist
Gewinnen des Faktor IX-Proteinprodukts,
wobei die Zelle das biologisch aktive Faktor IX-Proteinprodukt, gemessen in Relation zu dem Faktor IX Standard-Mononine, in einer Menge von mindestens etwa 15 mg/l produziert; und wobei die mindestens zwei Gene ein Gen enthalten, welches Vitamin-K abhängige Epoxid-Reduktase (VKOR) kodiert und ein Gen, welches Vitamin-K abhängige y-Glutamylcarboxylase (VKGC) kodiert.

2. Verfahren nach Anspruch 1, wobei mindestens eines der Gene über-exprimiert wird.

3. Verfahren nach Anspruch 2, wobei das überexprimierte Gen funktionsfähig an einen Chinese Hamster Elongationsfaktor 1-α (CHEF-1)-Promotor gekoppelt ist.

4. Verfahren nach Anspruch 1, wobei mindestens etwa 75% der Glutaminsäure-Reste innerhalb der Gla-Domäne des biologisch aktiven Faktor IX-Proteinprodukts Gammacarboxyliert sind.

5. Verfahren nach irgendeinem vorangehenden Anspruch, wobei mindestens 50%, mindestens 70% oder mindestens 80% des Faktor IX-Proteins biologisch aktiv ist.

6. Verfahren nach irgendeinem vorangehenden Anspruch, wobei das biologisch aktive Faktor IX Protein in einer Menge von mindestens etwa 20 mg/l produziert wird.

7. Verfahren nach irgendeinem vorangehenden Anspruch, wobei die Transfektion sequentiell ist und wobei die Transfektion der Säugetier-Zelle ferner Folgendes aufweist: Selektieren von Zellen, die hohe Gehalte an dem Faktor IX Proteinprodukt, VKOR oder VKGC exprimieren; Klonen der ausgewählten Zellen; und Gezieltes Vermehren bzw. Amplifizieren der geklonten Zellen.

8. Verfahren nach Anspruch 7, wobei der Schritt des Transfizierens mit mindestens zwei Genen durchgeführt wird, bevor der Schritt des Transfizierens mit dem Gen, das das Faktor IX-Protein kodiert, durchgeführt wird.

9. Verfahren nach Anspruch 7, wobei der Schritt des Transfizierens mit dem Gen, welches das Faktor IX-Protein kodiert, vor dem Schritt des Transfizierens mit den mindestens zwei Genen durchgeführt wird.

10. Verfahren nach einem der Ansprüche 7 bis 9, wobei die CHO-Zelle zur Expression endogener Spiegel eines oder mehrerer Verarbeitungsfaktoren vor der Transfektion ausgewählt wird.

11. Rekombinante CHO-Zelle, die ein Gen enthält, welches ein Faktor IX-Protein kodiert, welches funktionstüchtig an einen Chinese Hamster Elongationsfaktor 1-α (CHEF-1)-Promotor und mindestens zwei Gene, die funktionstüchtig an wenigstens einen Promotor gekoppelt sind, gekoppelt ist,
wobei die Zelle biologisch aktives F IX-Protein in einer Menge von mindestens 15 mg/l produziert, gemessen in Relation zu dem Faktor IX Standard-Mononine.
wobei die mindestens zwei Gene ein Gen aufweisen, welches Vitamin-K abhängige Epoxid-Reduktase (VKOR) kodiert und ein Gen, welches Vitamin-K abhängige γ-Glutamylcarboxylase (VKGC) kodiert.

12. Rekombinante Zelle nach Anspruch 11, wobei das mindestens eine Gen überexprimiert ist.

13. Rekombinante Zelle nach Anspruch 12, wobei das überexprimierte Gen-Produkt funktionsfähig an einen Chinese Hamster Elongationsfaktor 1-α (CHEF-1)-Promotor gekoppelt ist.

14. Verfahren zum Herstellen eines rekombinanten Faktor IX Proteinprodukts, welches folgende Schritte aufweist.
(a) Transfizieren einer CHO-Zelle (CHO = Chinese Hamster Ovary = Zelle aus dem Ovar des chinesischen Hamsters) mit einem Gen, welches das Faktor IX-Protein kodiert, welches funktionsfähig an einen Chinese Hamster Verlängerungs- bzw. Elongationsfaktor 1-α (CHEF-1 = Chinese Hamster elongation factor)-Promotor gekoppelt ist
(b) Auswählen von Zellen, welche hohe Spiegel des Faktor IX Proteinprodukts aufweisen;
(c) Transfizieren der ausgewählten Zellen mit mindestens zwei Genen, wobei die mindestens zwei Gene ein Gen enthalten, welches Vitamin-K abhängige Epoxid-Reduktase (VKOR) kodiert und ein Gen, welches Vitamin-K abhängige γ-Glutamylcarboxylase (VKGC) kodiert;
(d) Wiederholen von Schritt (b);
(e) optionales Wiederholen der Schritte (a) und/oder (c) gefolgt von (b);
(f) Klonen der ausgewählten Zellen;
(g) Züchten der geklonten Zellen; und
(h) Gewinnen des Faktor IX- Proteinproduktes aus den geklonten Zellen in einer Menge von mindestens 15mg/l.

## Revendications

1. Procédé pour fabriquer un produit de protéine Facteur IX biologiquement active recombinante, comprenant les étapes suivantes :
transfecter une cellule CHO avec un gène codant le produit de protéine Facteur IX lié fonctionnellement à un promoteur de facteur 1-α d'allongement de Hamster Chinois (CHEF-1), et au moins deux gènes liés fonctionnellement à au moins un promoteur, soit simultanément soit séquentiellement ; et
récolter le produit de protéine Facteur IX,
d'où il résulte que la cellule produit un produit de protéine Facteur IX active biologiquement, mesuré en référence à la mononine standard du Facteur IX, dans une quantité d'au moins 15 mg/litre ; et dans lequel lesdits au moins deux gènes comprennent un gène codant l'époxyde réductase dépendante de la vitamine K (VKOR), et un gène codant la y-glutamyl carboxylase dépendante de la vitamine K (VKGC).

2. Procédé selon la revendication 1, dans lequel au moins l'un des gènes est surexprimé.

3. Procédé selon la revendication 2, dans lequel le gène surexprimé est lié fonctionnement à un promoteur de facteur 1-α d'allongement de hamster Chinois (CHEF1).

4. Procédé selon la revendication 1, dans lequel au moins environ 75 % des résidus d'acide glutamique dans le domaine gla du produit de protéine Facteur IX biologiquement active sont gamma-carboxylés.

5. Procédé selon l'une quelconque des revendications précédentes, dans lequel au moins 50 %, au moins 70 % ou au moins 80 % des protéines Facteur IX sont biologiquement actives.

6. Procédé selon l'une quelconque des revendications précédentes, dans lequel la protéine Facteur IX biologiquement active est produite dans une quantité d'au moins 20 mg/litre.

7. Procédé selon l'une quelconque des revendications précédentes, dans lequel la transfection est séquentielle et dans lequel la transfection de la cellule mammaire comprend en outre : sélectionner des cellules qui expriment des niveaux élevés du produit de protéine Facteur IX, VKOR ou VKGC ; cloner les cellules sélectionnées ; et amplifier les cellules clonées.

8. Procédé selon la revendication 7, dans lequel l'étape de transfection avec lesdits au moins deux gènes est réalisée avant l'étape de transfection avec le gène codant la protéine Facteur IX.

9. Procédé selon la revendication 7, dans lequel l'étape de transfection avec le codage de gène de la protéine Facteur IX est réalisée avant l'étape de transfection avec lesdits au moins deux gènes.

10. Procédé selon l'une quelconque des revendications 7 à 9, dans lequel la cellule CHO est sélectionnée pour une expression de niveaux endogènes d'un ou plusieurs facteurs de traitement avant la transfection.

11. Cellule CHO recombinante, comprenant un gène codant une protéine Facteur IX lié fonctionnellement à un promoteur de facteur 1-α d'allongement de Hamster Chinois (CHEF-1) et au moins deux gènes liés fonctionnellement à au moins un promoteur,
dans laquelle la cellule produit une protéine FIX biologiquement active dans une quantité d'au moins 15 mg/litre, mesurée en référence à la mononine standard du Facteur IX, dans lequel lesdits au moins deux gènes comprennent un gène codant l'époxyde réductase dépendante de la vitamine K (VKOR), et un gène codant la γ-glutamyl carboxylase dépendante de la vitamine K (VKGC).

12. Cellule recombinante selon la revendication 11, dans laquelle au moins un gène est surexprimé.

13. Cellule recombinante selon la revendication 12, dans laquelle le gène surexprimé produit est lié fonctionnement à un promoteur de facteur 1-α d'allongement de Hamster Chinois (CHEF1).

14. Procédé pour produire un produit de protéine Facteur IX biologiquement active recombinante, comprenant les étapes suivantes : (a) transfecter une cellule CHO avec un gène codant la protéine Facteur IX lié fonctionnellement à un promoteur de facteur 1-α d'allongement de Hamster Chinois (CHEF1) ; (b) sélectionner des cellules qui expriment des niveaux élevés pour le produit de protéine Facteur IX ; (c) transfecter les cellules sélectionnées avec au moins deux gènes, lesdits au moins deux gènes comprenant un gène codant l'époxyde réductase dépendante de la vitamine K (VKOR), et un gène codant la γ-glutamyl carboxylase dépendante de la vitamine K (VKGC) ; (d) répéter l'étape (b) ; (e) optionnellement, répéter les étapes (a) et/ou (c) suivies de (b) ; (f) cloner les cellules sélectionnées ; (g) faire croître les cellules clonées ; et (h) récolter le produit de protéine Facteur IX à partir des cellules clonées dans une quantité d'au moins 15 mg/litre.
